**Europäisches Patentamt**

**European Patent Office**

**Office européen des brevets**

(19)

(11) Veröffentlichungsnummer : **0 095 170 B2**

(12) # NEUE EUROPÄISCHE PATENTSCHRIFT

(45) Veröffentlichungstag der neuen Patentschrift :
**05.02.92 Patentblatt 92/06**

(51) Int. Cl.⁵ : **C07G 17/00, B01D 61/14, B01D 61/48, // A61K35/14**

(21) Anmeldenummer : **83105046.3**

(22) Anmeldetag : **20.05.83**

---

(54) **Verfahren zur Gewinnung zellatmungsfördernder Wirkstoffe aus Kälberblut.**

---

(30) Priorität : **21.05.82 DE 3219248**

(43) Veröffentlichungstag der Anmeldung :
**30.11.83 Patentblatt 83/48**

(45) Bekanntmachung des Hinweises auf die Patenterteilung :
**16.04.86 Patentblatt 86/16**

(45) Bekanntmachung des Hinweises auf die Entscheidung über den Einspruch :
**05.02.92 Patentblatt 92/06**

(84) Benannte Vertragsstaaten :
**AT BE CH DE FR GB IT LI LU NL SE**

(56) Entgegenhaltungen :
**DE-A- 2 325 196
DE-B- 1 076 888
DE-C- 2 512 936**

(56) Entgegenhaltungen :
**GB-A- 824 375
US-A- 3 905 886
US-A- 4 043 896
H.STRATHMANN, "Trennung von molekularen Mischungen mit Hilfe synthetischer Membranen", Sternkopf Verlag, Darmstadt (DE), 1979; Seiten 9-10, 57-58, 76-77, 198-203
POLYMER SCIENCE & TECHNOLOGY, vol. 13, 1980; R.M.AHLGREN, Seiten 609-618**

(73) Patentinhaber : **Solco Basel AG
Gellertstrasse 18
CH-4052 Basel (CH)**

(72) Erfinder : **Wittenberger, Udo, Dr.
Mühleweg 17/6
CH-4133 Pratteln (CH)**

(74) Vertreter : **Spott, Gottfried, Dr. et al
Sendlinger-Tor-Platz 11
W-8000 München 2 (DE)**

EP 0 095 170 B2

## Beschreibung

Die Erfinfung betrifft ein Verfahren zur Gewinnung zellatmungsfördernder Wirkstoffe mit Molekullargewichten im Bereich von etwa 300 bis 8 000 Dalton durch Defibrinieren von Kälberblut unmittelbar nach der Entnahme durch intensives Rühren und Abfiltrieren des dabei gebildeten Fibrins, Hämolysieren der hierbei erhaltenen Lösung, Abtrennung der in der hämolysierten Lösung enthaltenen Proteine und Substanzen mit einem Molekulargewicht von über etwa 8 000 Dalton mittels eines Membrantrennverfahrens, Einengung der hierdurch erhaltenen, von Proteinen und höhermolekularen Substanzen befreiten Lösung unter vermindertem Druck bei einer 40 °C nicht übersteigenden Temperatur auf eine Dichte bei 20 °C im Bereich von 1,10 bis 1,15 g/ml und teilweise Abtrennung der im erhaltenen Konzentrat vorhandenen anorganischen Salze unter gegebenenfalls abschließender Verdünnung des erhaltenen Konzentrats zur Bildung einer isotonischen bis leicht hypertonischen Lösung mit einer Osmolarität im Bereich von 250 bis 550 mOsmol.

Die DE-B-10 76 888 beschreibt bereits ein Verfahren zur Gewinnung zellatmungsfördernder Wirkstoffe aus Blut, indem man beispielsweise frisches Kälberblut defibriniert, die erhaltene Lösung einer Hämolyse unterzieht, die hierdurch erhaltene hämolysierte Lösung zur Abtrennung der höhermolekularen Bestandteile, wie insbesondere von Proteinen, einer Dialyse unterwirft und das dabei anfallende Dialysat durch abschließende schonende Einengung auf einen Trockensubstanzgehalt von 30 bis 60 mg/ml in eine für therapeutische Zwecke direkt geeignete Wirkstofflösung überführt. Die bei diesem Verfahren erforderliche Dialyse kann den darin gemachten Angaben zufolge mit jedem bekannten und üblichen Dialysiermaterial durchgeführt werden, wobei der Einsatz von Zellophanschläuchen besonders geeignet sein soll.

Dieses bekannte Verfahren führt zwar zu einem Produkt mit der gewünschten therapeutischen Wirkung, hat jedoch den Nachteil, daß die hierzu notwendige Dialyse verhältnismäßig aufwendig und langwierig ist, ein bezüglich seiner oberen Molekulargewichtsgrenze und seiner Zusammensetzung Schwankungen unterworfenes und somit nicht gleichbleibend einheitliches Produkt ergibt und bezüglich der Produktausbeute zudem noch sehr zu wünschen übrig läßt.

Das Verfahren der DE-B-10 76 888 führt ferner auch zu einem Produkt mit einem verhältnismäßig hohen Gehalt an anorganischen Salzen, insbesondere an Natriumchlorid und Kaliumchlorid, was überwiegend auf eine Anreicherung des im Blut vorhandenen Anteils dieser Salze zurückzuführen ist, da sich solche Salze nach dem darin beschriebenen Verfahren nicht abtrennen lassen. Der Salzgehalt der hierbei anfallenden Wirkstoflösung kann beispielsweise etwa 25 bis 80 Gewichtsprozent ausmachen, so daß entsprechende Injektionslösungen oder hochdotierte topische Formen mehrfach hyperton sind. Infolge dieser Hypertonizität ist eine intramuskuläre Verabreichung solcher Wirkstofflösungen einerseits mit Schmerzen und andererseits mit einer Zellschädigung verbunden, die dem beabsichtigten Zellregenerationseffekt entgegenläuft.

Der störende zu hohe Salzgehalt der nach dem Verfahren der DE-B-10 76 888 und der auch nach der ersten Stufe des vorliegenden Verfahrens, nämlich der kontinuierlichen Ultrafiltration, erhältlichen zellatmungsfördernden Wirkstoffe ließe sich im Prinzip nach mehreren verschiedenen Methoden so erniedrigen, daß sich hierdurch direkt eine isotonische bis nur leicht hypertonische Wirkstofflösung ergibt, nämlich beispielsweise durch Dialyse, Ultrafiltration, Ionenaustausch oder Gelfiltration. Alle diese Verfahren sind für den vorliegenden Fall einer Teilentsalzung einer sehr komplex zusammengesetzten und aufgebauten Lösung mit einem Gehalt an zellatmungsfördernden Wirkstoffen jedoch entweder überhaupt nicht oder nur unter Erzielung sehr unbefriedigender Ergebnisse anwendbar. Gemäß DE-A-25 12 936 soll das Problem einer Entsalzung oder Teilentsalzung der in Rede stehenden zellatmungsfördernden Wirkstofflösung daher unter Anwendung eines ganz speziellen Gelfiltrationsverfahrens gelöst werden, nämlich durch Aufbringen der stark salzhaltigen Lösung der zellatmungsfördernden Wirkstoffe auf eine in einer perforierten Zentrifugentrommel befindliche Filtrationsschicht aus einem Gel mit hohem Vernetzungsgrad unter einem Verhältnis von Lösung zu Gelvolumen von etwa 1 : 2,5 bis 1 : 4,5. In dieser DE-A werden zugleich auch die Nachteile der anderen im Prinzip möglichen bekannten Verfahren zur Erzielung einer Teilentsalzung ausführlich diskutiert. Alle diese möglichen Verfahrenswege unter Einschluß des in DE-A 25 12 936 beschriebenen Verfahrens haben jedoch den Nachteil, daß ihre Trennleistung sehr gering und der apparative Aufwand sehr großt ist, da hier in der Regel in hoher Verdünnung gearbeitet werden muß. Ferner sind sie mit mehr oder weniger großen Wirkstoffverlusten verbunden, da neben der gewollten Abtrennung der anorganischen Salze auch eine Adsorption der zellatmungsfördernden Wirkstoffe an die benötigten Trennmittel stattfindet. Die dabei zwangsläufig erforderliche Regenerierung der zu verwendenden Gele und Ionenaustauscherharze erfordert zudem einen großen Zeitaufwand und ermöglicht lediglich eine diskontinuierliche Arbeitsweise unter ständiger Änderung der Trennbedingungen.

Das aus DE-B-10 76 888 bekannte Verfahren zur Gewinnung zellatmungsfördernder Wirkstoffe hat obigen Angaben zufolge die verschiedensten Mängel, und diese können auch nicht unter zusätzlicher Heranziehung des in DE-A-25 12 936 beschriebenen Verfahrens zur Abtrennung des zu hohen Gehalts an störenden anorganischen Salzen gelöst werden. Aufgabe der Erfindung ist daher die Schaffung eines neuen Verfahrens zur Gewinnung zellatmungsfördernder Wirkstoffe der in Rede stehenden Art, das die Nachteile der bekannten Arbeitsweisen nicht kennt und vor allem einfach und kontinulerlich durchgeführt werden kann, sauber steuerbar ist und hierdurch ein Produkt mit einheltlicher oberer und in gewissem Ausmaß auch unterer Molekulargewichtsbegrenzung ergibt und ferner durch die Möglichkeit einer gesteuerten Teilentsalzung direkt zu einer Lösung der zellatmungsfördernden Wirkstoffe führt, die der jeweiligen Verabreichungsart angepaßt ist und sowohl bei topischer als such bei systemischer Verabreichung eine bessere physiologische Verträglichkeit als das in bekannter Weise erhältliche therapeutische Produkt mit zellatmungsfördernder Aktivität aufweist.

Diese Aufgabe wird beim Verfahren der eingangs angegebenen Art erfindungsgemäß nun dadurch gelöst, daß als Membrantrennverfahren eine kontinuierliche mehrstufige Ultrafiltration unter Verwendung von Membranen mit einer Molekulargewichtsausschlußgrenze von über etwa 8 000 Dalton angewandt wird und die teilweise Abtrennung der anorganischen Salze durch Elektrodialyse mittels eines Zellpakets aus alternierend angeordneten Kationen- und Anionenaustauschermembranen mit einer Membrandurchlässigkeit bis zu einem Molekulargewicht von etwa 300 Dalton und unter Anwendung eines Stromdichtebereichs von 5 bis 70 mA/cm² freiem Membranquerschnitt, vorzugsweise 20 bis 50 mA/cm² freiem Membranquerschnitt, einer Gleichspannung von 0,2 bis 2 V pro Membran, vorzugsweise 0,5 bis 1 V pro Membran, und eines Temperaturbereiches von 0 bis 30 °C durchgeführt wird.

Die erfindungsgemäße Lösung besteht demnach in einer Kombination aus einem Ultrafiltrationsverfahren zur Abtrennung der in der hämolysierten Lösung enthaltenen Proteine und Substanzen mit höherem Molekulargewicht und einer Elektrodialyse zur teilweisen Abtrennung der störenden anorganischen Salze.

Vorzugsweise wird das erfindungsgemäße Verfahren so durchgeführt, daß bei der Ultrafiltration Membranen mit einer Molekurlargewichtsausschlußgrenze von über etwa 5 000 Dalton und bei der Elektrodialyse Membranen mit einer Durchlässigkeit bis zu einem Molekulargewicht von etwa 400 Dalton verwendet werden.

Es hat sich als besonders vorteilhaft erwiesen, wenn man bei der Stufe der erfindungsgemäßen Ultrafiltration in die zwischen der ersten und der letzten Stufe der mehrstufigen Ultrafiltration liegenden Stufen jeweils eine solche Menge an Verdünnungsmittel einführt, daß hierdurch die aus dem jeweiligen Ultrafiltrationsmodul auslaufende Menge an Ultrafiltrat unter Beibehaltung eines etwa konstanten Niveaus an ultrazufiltrierender Blutlösung kompensiert wird, wobei es besonders günstig ist, wenn man in allen von der letzten Stufe liegenden Stufen der mehrstufigen Ultrafiltration jeweils unter Beibehaltung eines etwa konstanten Niveaus an ultrazufiltrierender Blutlösung arbeitet.

Die zweite Stufe des erfindungsgemäßen Verfahrens, nämlich die mehrstufige Elektrodialyse, wird vorzugsweise unter Bildung einer Lösung mit einer Leitfähigkeit im Bereich von 40 bis 75 mS/cm und mit einer Dichte bei 20 °C im Bereich von 1,05 bis 1,08 g/ml durchgeführt.

Das erfindungsgemäße Verfahren führt zu einem therapeutischen Produkt, dessen zellatmungsfördernde Wirdung im wesentlichen die gleiche ist wie die des nach DE-B-10 76 888 erhältlichen Produkts, stellt jedoch durch die Anwendung der Kombination aus Ultrafiltration und Elektrodialyse eine wesentliche Verbesserung in mancherlei Hinsicht dar. Die Ultrafiltration ergibt ein einheitliches Produkt unter sauberer Abtrennung der Proteine und sonstigen höhermolekularen Substanzen mit dem gleichzeitigen Vorteil einer besseren Wirtschaftlichkeit hinsichtlich Zeitbedarf, Platzbedarf und Ausbeute. Die sich daran nach einer gewissen Einengung der bei der Ultrafiltration erhaltenen Lösung anschließende Elektrodialyse hat den Vorteil, daß sie einerseits unter Verwendung einer verhältnismäßig konzentrierten Lösung, beispielsweise einer Lösung mit einem Feststoffgehalt von 15 bis 30 Gewichtsprozent, durchgeführt werden kann, und daß hierbei zudem in einem quasi stationären Gleichgewicht gearbeitet werden kann, das sich nach einer Anfangsphase an den Ionenaustauschermembranen einstellt. Durch Veränderung der Parameter Stromdichte, Temperatur und Zeit läßt sich das Entsalzungsverfahren ferner in bestimmte Richtungen steuern und derart optimieren, daß nur ein Minimum an organischer Substanz verlorengeht. Der Grad der jeweiligen Entsalzung wird am einfachsten durch kontinuierliche Messung der Leitfähigkeit oder auch durch kontinuierliche Bestimmung der Osmolarität ermittelt.

Die beim vorliegenden Verfahren erforderlichen bekannten oder erfinderischen Maßnahmen werden im übrigen wie folgt durchgeführt.

Das als Ausgangsmaterial benötigte Kälberblut wird unmittelbar nach seiner Entnahme durch intensives Rühren, gegebenenfalls unter Abkühlung, und durch anschließendes Abfiltrieren des dabei gebildeten Fibrins defibriniert. Dieser Vorgang wird gewöhnlich direkt in den entsprechenden Schlachthöfen durchgeführt.

Nach erfolgter Defibrinierung wird das Blut, gegenenenfalls nach Versetzen mit einem Konservierungsmittel, unmittelbar eingefroren und bis zu seiner weiteren Verwendung in gefrorenem Zustand gelagert.

Zur Freisetzung der zellatmungsfördernden Wirkstoffe muß das defibrinierte und zur Lagerung gegebenenfalls eingefrorene Blut einer Hämolyse unterzogen werden, nämlich einer Auflösung der roten Blutkörperchen. Zu diesem Zweck müssen die Zellmembranen der Blutkörperchen in irgendeiner Weise zerstört werden.

Eine solche Zerstörung läßt sich sowohl auf chemischem als auch auf mechanischem Weg erreichen. Zur chemischen Hämolyse versetzt man das defibrinierte Blut beispielsweise mit Fermenten oder Bakterien, die die Zellmembranen angreifen und zerstören. Die mechanische Hämolyse kann entweder durch Zugabe von Mitteln erfolgen, die zu einer Erhöhung des osmotischen Drucks im Zellinneren führen, wie beispielsweise durch Zusatz von Wasser oder organischen Lösungsmitteln, wie Ethanol, Diethylether oder Aceton, wodurch die Zellmembranen dann zerplatzen, oder sie läßt sich auch durch sogenannte Eishämolyse erreichen, indem man das Blut derart langsam einfriert, daß sich die Zellmembranen durchstoßende größere Eiskristalle ausbilden können. Nach erfolgter Hämolyse sind praktisch alle roten Blutkörperchen zerstört und die darin enthaltenen Stoffe freigesetzt.

Die Hämolyse wird zweckmäßigerweise ebenfalls in Gegenwart von Konservierungsmitteln durchgeführt.

Das hierdurch erhaltene Blut ist dann fertig zur Durchführung der ersten erfindungsgemäßen Stufe des vorliegenden Verfahrens, nämlich der kontinuierlichen mehrstufigen Ultrafiltration unter Verwendung von Membranen mit einer Molekulargewichtsausschlußgrenze von über etwa 8 000 Dalton, und vorzugsweise von über etwa 5 000 Dalton.

Die bei der Ultrafiltration verwendeten Membranen können aus den verschiedensten Materialien bestehen, wobei Membranen aus Cellulosetriacetat oder hydrophilem Polyolefin bevorzugt sind.

Membranen dieser Art mit einer Molekulargewichtsausschlußgrenze von über etwa 8 000 Dalton sind hinsichtlich ihrer Durchlässigkeit gekennzeichnet durch eine 100%-ige Abtrennung von Dextran mit einem Molekulargewicht von 20 000, eine nominal 85 %-ige Abtrennung von Dextran mit einem Molekulargewicht von 10 000, eine nominal 50 %-ige Abtrennung von Polyethylenglykol mit einem Molekulargewicht von 6 000 und eine nominal 10%-ige Abtrennung von Lactose mit einem Molekulargewicht von 342.

Membranen mit einer Molekulargewichtsausschlußgrenze von über etwa 5 000 Dalton sind bezüglich ihrer Durchlässigkeit gekennzeichnet durch eine 100%-ige Abtrennung von Dextran mit einem Molekulargewicht von 10 000, eine nominal 97 %-ige Abtrennung von Polyethylenglykol mit einem Molekulargewicht von 6 000 und eine nominal 30 %-ige Abtrennung von Polyethylenglykol mit einem Molekulargewicht von 1000.

Die Ultrafiltration der nach Defibrinieren und Hämolysieren von Kälberblut erhaltenen Lösung unter Verwendung von Membranen der oben beschriebenen Art ergibt dann ein fertiges Produkt mit einer oberen Molekulargewichtsgrenze von etwa 8 000 Dalton oder von etwa 5 000 Dalton.

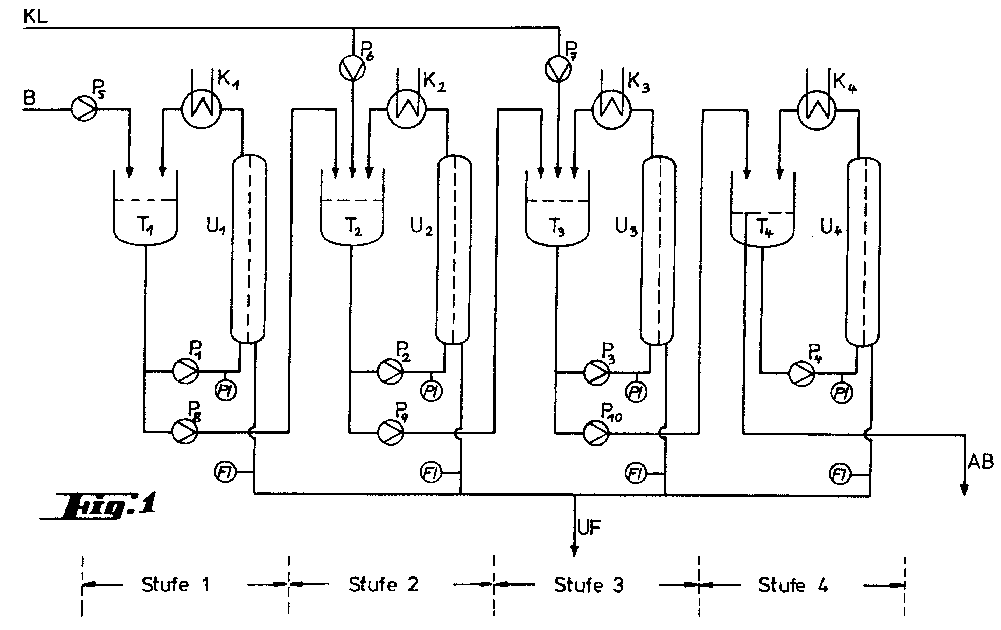

Zur Durchführung der kontinuierlichen mehrstufigen Ultrafiltration wird vorzugsweise eine aus vier Stufen bestehende Ultrafiltrationsanlage verwendet, wie sie später im Beispiel zusammen mit der Figur 1 näher beschrieben wird. Eine solche Anlage enthält Ultrafiltrations-Module mit der Typenbezeichnung B1, wie sie im Firmenprospekt BPL 3/73 2M von Paterson Candy International Ltd., Reverse Osmosis Division, Whitchurch, Großbritannien, näher beschrieben sind und auch aus DE-A-2 065 812 hervorgehen. Diese spezielle Anlage enthält Membranen mit einer Molekulargewichtsausschlußgrenze von über etwa 8000 Dalton. Sie kann genausogut jedoch auch mit Membranen mit einer anderen Molekulargewichtsausschlußgrenze ausgerüstet werden, beispielsweise einer Molekulargewichtsausschlußgrenze von über etwa 5 000 Dalton.

Die Ultrafiltrationsanlage wird unter Anwendung von Drücken von beispielsweise 8 bis 10 bar und Kühlen der zu ultrafiltrierenden Lösung auf eine Temperatur von beispielsweise 18 bis 22 °C betrieben. Sie wird vorzugsweise mit einer Lösung von Kälberblut versorgt, die mit einem üblichen konservierungsmittel versetzt ist, beispielsweise einer alkoholischen Lösung von 4-Hydroxybenzoesäuremethylester oder 4-Hydroxybenzoesäurepropylester, wobei eine ethanolische Lösung aus einer Kombination dieser beiden Konservierungsmittel bevorzugt wird.

Zwischen der ersten Stufe und der letzten Stufe der kontinuierlichen mehrstufigen Ultrafiltration wird zweckmäßigerweise als Verdünnungsmittel für die im jeweiligen Ultrafiltrationsmodul anfallende konzentrierte und dann erneut ultrazufiltrierende Blutlösung jeweils eine solche Menge an Konservierungsmittel eingeführt, daß hierdurch die aus dem jeweiligen Ultrafiltrationsmodul auslaufende Menge an Ultrafiltrat unter Beibehaltung eines etwa konstanten Niveaus an ultrazufiltrierender Blutlösung kompensiert wird. Zweckmäßigerweise wird in allen vor der letzten Stufe liegenden Stufen bei der kontinuierlichen mehrstufigen Ultrafiltration jeweils unter Beibehaltung eines etwa konstanten Niveaus an ultrazufiltrierender Blutlösung gearbeitet. In der letzten Stufe wird dann ohne jegliche weitere Verdünnung die aus der vorletzten Stufe kommende Blutlösung ausdialysiert und somit ausgepreßt.

Das bei der Ultrafiltration schließlich anfallende Abfallblut wird verworfen.

Die bei der Ultrafiltration als Ultrafiltrat erhaltene, von Proteinen und höhermolekularen Substanzen befreite Lösung wird in einer weiteren Stufe unter vermindertem Druck bei einer 40 °C nicht übersteigenden Temperatur auf eine Dichte bei 20 °C im Bereich von 1,10 bis 1,15 g/ml eingeengt, indem man diese Lösung unter vermindertem Druck bei einer Temperatur von beispielsweise 30 bis 35 °C destilliert. Zweck dieser Einengung ist einmal die Entfernung des gegebenenfalls zur Hämolyse verwendeten organischen Lösungsmittels oder des für das jeweilige Konservierungsmittel benötigten organischen Lösungsmittels unter gleichzeitiger Abtrennung des Konservierungsmittels und zum anderen Mal eine Konzentrierung der ultrafiltrierten Lösung auf einen zur Durchführung der anschließenden Stufe der erfindungsgemäßen Elektrodialyse geeigneten Trockengehalt, nämlich beispielsweise einen Trockengehalt von 180 bis 230 mg/ml. Das bei dieser Einengung ausfallende Konservierungsmittel wird am besten durch Filtrieren abgetrennt, wobei die in der Lösung eventuell noch vorhandenen Restmengen an Konservierungsmittel durch geeignete Einstellung des pH-Wertes der Lösung, beispielsweise durch Zusatz kleiner Mengen konzentrierter Salzsäure bis zu einem pH-Wert von 5, ausgefällt und dann abfiltriert werden. Die für diese Einengung erforderliche Destillation wird zweckmäßigerweise in einem geeigneten Vakuumverdampfer durchgeführt.

Das nach obiger Einengung erhaltene Konzentrat unterzieht man dann zur teilweisen Abtrennung der darin enthaltenen anorganischen Salze und Bildung einer isotonischen bis leicht hypertonischen Lösung mit einer Osmolarität im Bereich von 250 bis 550 mOsmol oder einer Leitfähigkeit im Bereich von 40 bis 75 mS/cm oder einer Dichte bei 20 °C im Bereich von 1,05 bis 1,08 g/ml der zweiten erfindungsgemäßen Verfahrensstufe, nämlich der Stufe der Elektrodialyse mittels eines Zellpakets aus alternierend angeordneten Kationen- und Anionenaustauschermembranen mit einer Membrandurchlässigkeit bis zu einem Molekulargewicht von etwa 300 Dalton, vorzugsweise bis zu einem Molekulargewicht von etwa 400 Dalton, unter Anwendung eines Stromdichtebereiches von 5 bis 70 mA/cm² freiem Membranquerschnitt, vorzugsweise 20 bis 50 mA/cm² freiem Membranquerschnitt, einer Gleichspannung von 0,2 bis 2 V pro Membran, vorzugsweise 0,5 bis 1 V pro Membran, und eines Temperaturbereiches von 5 bis 30 °C.

Die hierzu verwendeten Membranen sind gekennzeichnet durch eine Durchlässigkeit für anorganische und organische Ionen mit einem Molekulargewicht bis zu etwa 300 oder vorzugsweise bis zu etwa 400, während diese Membranen für Oligopeptide und größere Moleküle gesperrt sind.

Unter Verwendung solcher Membranen ergibt sich für die Wirkstoffe eine untere Molekulargewichtsgrenze von etwa 300 Dalton oder vorzugsweise von etwa 400 Dalton.

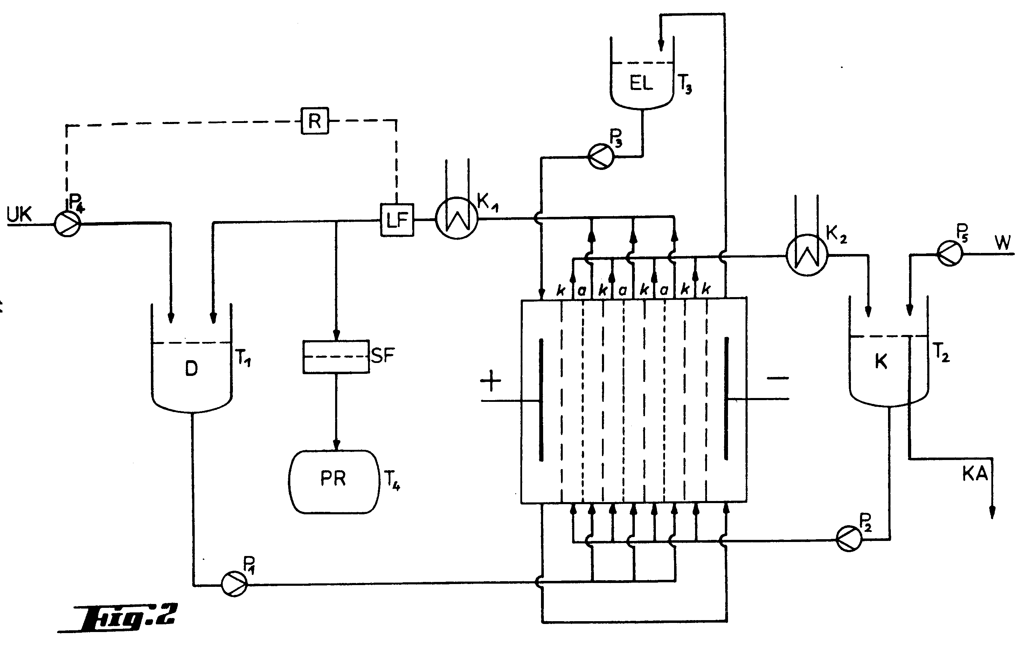

Die Stufe der vorliegenden Elektrodialyse läßt sich unter Einsatz üblicher Elektrodialyseanlagen aus einem Zellpaket aus alternierend angeordneten Kationen- und Anionenaustauschermembranen mit der jeweils gewünschten Membrandurchlässigkeit durchführen. Im Beispiel wird hierzu eine Elektrodialyseanlage gemäß der später näher beschriebenen Figur 2 verwendet. Hierbei handelt es sich um eine Anlage mit der Typenbezeichnung BEL2 von Berghof GmbH, Tübingen, Deutschland. Die Membranen dieser Anlage weisen eine Membrandurchlässigkeit bis zu einem Molekulargewicht von etwa 400 Dalton auf. Eine ausführliche Beschreibung des Zellpakets dieser Elektrodialyseanlage geht aus DE-A 2 946 284 hervor.

Die beim erfindungsgemäßen verfahren anfallende wässrige Lösung mit einem Gehalt an zellatmungsfördernden Wirkstoffen kann je nach der für sie beabsichtigten Anwendungsform entweder als solche direkt zu therapeutischen Zwecken eingesetzt werden, gewünschtenfalls mit pyrogenfreiem Wasser weiter verfünnt werden oder bedarfsweise auch noch weiter konzentriert werden. Die entsprechenden Arzneimittelformem stellen dann Beispielsweise Lösungen, Aerosole, Salben, Cremes oder Gele dar. Die zellatmungsfördernde Wirkung solcher Arzneimittelformen ist — wie bereits angegeben — im wesentlichen die gleiche wie die der nach DE-B-1 076 888 erhältlichen Wirkstoff. Demnach werden solche Arzeiformen insbesondere zur Beschleunigung und Beeinflussung von Heilungsprozessen verwendet. Die hierbei anzuwendende Wirkstoffdosierung ist abhängig vom jeweiligen Anwendungsmodus sowie von der Natur und der Schwere des zu behandelnden Zustandes. Wundarten, die sich mit den nach dem vorliegenden Verfahren gewinnbaren zellatmungsfördernden Wirkstoffen behandeln lassen, sind beispielsweise Brandwunden, Ulzera oder Dekubitus. Der Wirkstoff kann dabei intravenös, intraarteriell oder intramuskulär und — im Falle offener Wunden — auch topisch angewandt werden. Für eine Injektionstherapie, wie zur Behandlung von Ulzera beim Menschen, ist beispielsweise anfangs eine intravenöse oder intraarterielle Tagesdosis von 200 bis 800 mg Wirksubstanz erforderlich, und hieran schließt sich eine intravenöse oder intramuskuläre Nachbehandlung mit Tagesdosen von 80 bis 200 mg Wirdstoff an.

Bezüglich dieser und anderer Anwendungsmöglichkeiten eines solchen Wirkstoffs wird allgemein auf die Broschüre "Solcoseryl reaktiviert den gestörten Energiestoffwechsel der Zelle, regeneriert das Gewebe" der Solco Basel AG, Birsfelden, Schweiz, mit der Veröffentlichungsnummer SS-CH/d-6.81, hingewiesen.

Wesentliche Voraussetzung für die erfolgreiche Anwendbarkeit des erfindungsgemäßen Verfahrens ist, daß es hierbei, und zwar insbesondere bei der Ultrafiltration und vor allem bei der Elektrodialyse, zu keiner Beeinträchtigung der biologischen Aktivität des Kälbertblutextrakts kommen darf. Es muß also dessen ursprüngliche biologische Aktivität voll erhalten bleiben. Demnach soll das erfindungsgemäße Verfahren auch zu einem Blutextrakt führen, dessen biologische Aktivität wenigstens der des nach DE-B-1 076 888 erhältlichen Produkts entspricht oder diesem gegenüber sogar höher ist.

Die Gefahr eines möglichen Verlustes an biologischer Aktivität während der erfindungsgemäß durchzuführenden Ultrafiltration ist hierbei als gering anzusehen, da bei dieser Verfahrensstufe lediglich Proteine und höhermolekulare Substanzen abgetrennt werden und der Hauptteil an biologischer Aktivität entsprechenden Untersuchungen zufolge im unteren Molekulargewichtsbereich der nach dem vorliegenden Verfahren gewinnbaren zellatmungsfördernden Wirkstoffe liegen dürfte. Es kann zudem davon ausgegangen werden, daß die Ultrafiltration im wesentlichen keine andere Beeinflussung des Blutextrakts ergibt, als die nach dem Verfahren der DE-B-1 076 888 durchzuführende einfache Dialyse. Eine vergleichende Untersuchung der möglichen Beeinträchtigung der biologischen Aktivität durch die erfindungsgemäße Ultrafiltration kann daher unterbleiben.

Die sich an die Ultrafiltration anschließende Einengung der wirkstoffhaltigen Lösung wird ebenfalls unter Bedingungen durchgeführt, die mit keiner Beeinträchtigung der biologischen Aktivität dieses Blutextrakts verbunden sein können. Auch bei dieser Stufe können demnach vergleichende Untersuchungen entfallen.

Die erfindungsgemäße Abtrennung der im Wirkstoffkonzentrat vorhandenen anorganischen Salze mittels Elektrodialyse könnte dagegen zu einer störenden Beeinträchtigung der biologischen Aktivität des Blutextrakts führen. Hierbei müssen nämlich verhältnismäßig große Salzmengen abgetrennt werden, und die Molekulargewichte der abzutrennenden Salze liegen zudem verhältnismäßig nahe an der unteren Molekulargewichtsgrenze der erfindungsgemäß zu gewinnenden zellatmungsfördernden Wirkstoffe. Es muß daher anhand entsprechender Untersuchungen festgestellt werden, ob sich durch die Stufe der Elektrodialyse bei der hiernach erhältlichen Wirkstofflösung gegenüber dem Ausgangskonzentrat eine Beeinträchtigung der biologischen Aktivität ergibt. Weiter ist natürlich zu untersuchen, wie sich die physikalischen Eigenschaften durch Anwendung dieser Elektrodialyse gegenüber dem Ausgangskonzentrat verändern und welche wesentlichen Stoffe neben den anorganischen Salze hierdurch abgetrennt werden.

Die vergleichenden Untersuchungen über das jeweilige Ausmaß an biologischer Aktivität sind nach drei verschiedenen Methoden durchgeführt worden, nämlich durch

a) Prüfung der wachstumsfördernden Wirkung bei Fibroblasten nach Schädigung durch Carbonatentzug (Ermittlung der Fibroblasten-Aktivität),

b) Prüfung der $O_2$-stoffwechselsteigernden Wirkung nach Warburg (Ermittlung der Warburg-Aktivität) und

c) Prüfung der wundheilungsfördernden Wirkung bei standardisierten, dorsalen Brandwunden von Ratten (Ermittlung der Wundheilungsdauer gegen physiologische Kochsalzlösung).

Die beiden ersten in-vitro-Untersuchungen ergaben unter allen Versuchsbedingungen, daß die volle biologische Aktivität erhalten bleibt und teilweise sogar erhöht wird, da die zellschädigenden Salze nicht mehr vorhanden sind. Die Wundheilungsuntersuchungen zeigten das erwartete Ergebnis, daß die nach dem vorliegenden Verfahren erhältlichen teilentsalzten Blutextrakte von den Versuchstieren besser vertragen werden.

Bei allen Vergleichen der Aktivität zwischen dem nichtentsalzten Ausgangskonzentrat und dem teilentsalzten Extrakt wurde selbstverständlich unter normierten Verhältnissen gearbeitet, nämlich unter Verwendung von Lösungen mit gleichem Gehalt an organischem Wirkstoff, und nicht mit gleichem Trockengehalt. Zu diesem Zweck wurden die zu vergleichenden Lösungen jeweils auf einen bestimmten Gehalt an Gesamtstickstoff verdünnt, beispielsweise auf einen Stickstoffgehalt von 1 mg pro ml Injektionslösung, und dieser Gesamtstickstoffgehalt wurde dann stellvertretend für den in den jeweiligen Lösungen vorhandenen Anteil an organischem Material genommen. Genauso wurde natürlich auch bei den für therapeutische Zwecke verwendeten Lösungen vorgegangen, wobei jede Charge zusätzlich im Warburg-Versuch und im Fibroblasten-Versuch überprüft wurde.

Anhand vergleichender Untersuchungen wurden die Veränderungen bezüglich Trockengehalt, Leitfähigkeit, Osmolarität und Aschegehalt ermittelt. Die sich durch die Abtrennung der niedermolekularen Substanzen, nämlich vorwiegend der anionischen Ionen und anderer niedermolekularer Produkte, gegenüber dem Ausgangskonzentrat ergebenden Veränderungen wurden durch Vergleich der chemischen Analysen hierfür charakteristischer Stoffe und Parameter ermittelt.

Die bei allen vergleichenden Untersuchungen unter Verwendung von zwei verschiedenen Ausgangskonzentraten und acht erfindungsgemäß gewonnenen teilentsalzten Extrakten erhaltenen Ergebnisse gehen aus Tabelle I des Beispiels hervor. Sie belegen. daß neben den anorganischen Ionen teilweise auch niedere Carbonsäuren und Aminosäuren entfernt werden, die biologische Aktivität jedoch voll erhalten bleibt.

Zum weiteren Nachweis, daß die erfindungsgemäß durchzuführende Elektrodialyse keine oder eine nur unwesentliche Veränderung der Zusammensetzung des Blutextrakts bezüglich seiner organischen Komponenten ergibt, wurden auch hochdrucklüssigkeitschromatographische Untersuchungen durchgeführt. Hierbei ergaben sich für die Ausgangskonzentrate und die teilentsalzten Extrakte jeweils identische Chromatogramme.

Das erfindungsgemäße Verfahren wird im folgenden anhand eines Beispiels weiter erläutert.

Beispiel

Ultrafiltration

Es wird eine Ultrafiltrationsanlage der eingangs erwähnten Art (B1 von Paterson Candy International Ltd.)

verwendet. Diese vierstufige Ultrafiltrationsanlage und ihr Betrieb gehen schematisch aus Figur 1 hervor.

Jede Stufe der insgesamt vierstufigen Anlage (Stufen 1 bit 4) ist mit zwei Ultrafiltrationsrohrmodulen ($U_1$ bis $U_4$) zu je 1,7 m² Filterfläche bestückt. Ihre Membranen bestehen aus Celluloseacetat mit der bereits erwähnten Durchlässigkeitschrakteristik, nämlich mit einer Molekulargewichtsausschlußgrenze von über etwa 8 000 Dalton. Die Druckförderpumpen $P_1$ bis $P_4$ sind Drehkolbenpumpen, die bei einem Druck von 10 bar eine Leistung von etwa 1 000 l/h erbringen. Die Zulaufpumpen $P_5$ bis $P_7$ und die Ablaufpumpen $P_8$ bis $P_{10}$ sind Dosierpumpen, die in einem Bereich bon 2 bis 20 l/h frei eingestellt werden können. Die insgesamt vier Tanks $T_1$ bis $T_4$ fassen jeweils etwa 300 l. Die in sie eingespeilsten Lösungen werden über die kühlwassergespeisten Kühler $K_1$ bis $K_4$ auf einer Temperatur von 18 °C gehalten. Mit PI sind jeweils Druckanzeigegeräte bezeichnet, während mit FI* jeweils Durchflußanzeigegeräte bezeichnet sind. Die darin weiter enthaltenen Ankürzungen haben folgende Bedeutungen :

B = Defibrinierte und hämolysierte Blutlösung

KL = Konservierungsmittellösung

UF = Ultrafiltrat

AB = Abfallblut

Zum Anfahren der Anlage legt man in den Tanks $T_1$ (Stufe 1), $T_2$ (Stufe 2) und $T_3$ (Stufe 3) jeweils 250 l defibriniertes und hämolysiertes Kälberblut B vor, das als Konservierungsmittelösung 15 Volumenprozent einer 2%-igen (Gewicht/Volumen) alkoholischen Lösung eines 9 : 1-Gemisches (Gewicht/Gewicht) aus 4-Hydroxybenzoesäuremethylester und 4-Hydroxybenzoesäurepropylester enthält. Sodann werden die Druckförderpumpen $P_1$ bis $P_3$ in Gang gesetzt, wodurch das Ultrafiltrat UF auszulaufen beginnt und sich in gleichem Maße auch der Inhalt der Tanks $T_1$ bis $T_3$ verringert. Sobald der Inhalt der Tanks $T_1$ bis $T_3$ etwa 150 l beträgt, werden die Zulaufpumpen $P_5$ bis $P_7$ und die Ablaufpumpen $P_8$ bis $P_{10}$ in Gang gesetzt. Über die Zulaufpumpe $P_5$ werden dann 10 l/h konservierungsmittelhaltiges defibriniertes und hämolysiertes Kälberblut der oben bereits beschriebenen Art eingespeist. Gleichzeitig werden über die Zulaufpumpen $P_6$ and $P_7$ in die Tanks $T_2$ und $T_3$ als Verdünnungsmittel jeweils 10 l/h einer Konservierungsmittellösung KL aus 85 Volumenprozent Wasser und 15 Volumenprozent einer 1%-igen (Gewicht/Volumen) alkoholischen Lösung eines 9 : 1-Gemisches (Gewicht/Gewicht) aus 4-Hydroxybenzoesäuremethylester und 4-Hydroxybenzoesäurepropylester eingeführt. Die Ablaufpumpen $P_8$ bis $P_{10}$ werden so einreguliert, daß das Niveau in den Tanks $T_1$ bis $T_3$ konstant bei 150 l bleibt. In die Tanks $T_2$ und $T_3$ wird dabei jeweils eine solche Menge an Verdünnungsmittel KL eingespeist, daß hierdurch die jeweils auslaufende Menge an Ultrafiltrat UF unter Beibehaltung eines konstanten Niveaus an ultrazufiltrierender Blutlösung kompensiert wird.

Sobald im Tank $T_4$ (Stufe 4) etwa 120 l Lösung aus der Stufe 3 vorhanden sind, wird die Druckförderpumpe $P_4$ eingeschaltet. Das aus dem Überlauf dieses Tanks $T_4$ austretende Abfallblut AB wird verworfen.

Nach etwa 36 Stunden hat sich in allen Stufen ein Gleichgewicht eingestellt. Das aus den Ultrafiltrationsrohrmodulen $U_1$ bis $U_4$ auslaufende Ultrafiltrat UF ist somit für das vorliegende Verfahren repräsentativ, so daß es für die weitere Verwendung (nachfolgende Einengung) gesammelt wird. Der Auslauf an Ultrafiltrat UF beträgt bei der Stufe 1 etwa 4,7 l/h, bei der Stufe 2 etwa 11 l/h, bei der Stufe 3 etwa 10,4 l/h hund bei der Stufe 4 etwa 1,8 l/h. Die Fördermengen an Ultrafiltrat UF betragen demnach 5,3 l/h für die Ablaufpumpe $P_8$, 4,3 l/h für die Ablaufpumpe $P_9$ und 3,9 l/h für die Ablaufpumpe $P_{10}$, während der Überlauf an Abfallblut AB aus dem Tank $T_4$ (Stufe 4) 2,1 l/h ausmacht.

Einengung

Das bei der obigen vierstufigen Ultrafiltration erhaltene Ultrafiltrat UV wird dann unter einem verminderten Druck von 30 mbar und bei einer Temperatur von 35 °C durch Destillation auf eine Dichte bei 20 °C von 1,130 mg/ml eingeengt und hierdurch von dem in der Konservierungsmittellösung enthaltenen Alkohol befreit. Bei dieser Einengung fällt auch der Großteil des Konservierungsmittels aus, der abfiltriert wird. Das erhaltene Filtrat wird in kleinen Anteilen mit konzentrierter Salzäure bis zum Erreichen eines pH-Wertes von 5 versetzt, wodurch die restlichen Mengen an Konservierungsmittel ausfallen. Dieser Niederschlag wird wiederum abfiltriert.

Das unter Anwendung der obigen Ultrafiltration und Einengung erhaltene Material wird zusammengefaßt und dient dann als Ausgangskonzentrat mit der Bezeichnung Nr. 733.10 für die sich daran anschließende erfindungsgemäße Stufe der Elektrodialyse.

Wiederholung von Ultrafiltration und Einengung

Die Beiden obigen Verfahrensstufen werden zu einem späteren Zeitpunkt unter Verwendung von Kälberblut anderer Herkunft im einzelnen genau wiederholt, wodurch man zu einem weiteren Ausgangskonzentrat mit der Bezeichnung Nr. 350 03 für die sich dann anschließende Stufe der Elektrodialyse gelangt.

Elektrodialyse

Die in obiger Weise erhaltenen Ausgangskonzentrate Nr. 733 10 und Nr. 350 03 werden einmal in zwei Teilmengen und zum anderen Mal in sechs Teilmengen unterteilt und in jeweils getrennten Arbeitsvorgängen der aus der Figur 2 hervorgehenden Elektrodialyseanlage zugeführt. Auf diese Weise gelangt man dann unter Verwendung des Ausgangskonzentrats Nr. 733 10 zu den Elektrodialysaten Nr. 12 und Nr. 13 und unter Einsatz des Ausgangskonzentrats Nr. 350 03 zu den Elektrodialysaten Nr. 14, Nr. 15, Nr. 16, Nr. 17, Nr. 18 und Nr. 19.

Es wird eine Elektrodialyseanlage der eingangs erwähnten Art (BEL2 von Berghof GmbH) verwendet. Diese Anlage und ihr Betrieb gehen schematisch aus Figur 2 hervor.

Kernstück dieser Anlage ist ein Zellpaket, das aus sieben Paaren aus je einer Anionenaustauschermembran a und je einer Kationenaustauschermembran k (Typ CMV und AMV) besteht und an jeder Seite als Abschluß zusätzlich noch eine Membrane k enthält. (In der Schemazeichnung sind aus Vereinfachungsgründen nur drei Ionenaustauscherpaare eingezeichnet.) Die verwendeten Membrane weisen eine Durchlässigkeit bis zu einem Molekulargewicht von etwa 400 auf.

Am Zellpaket sind folgende drei Flüssigkeitskreisläufe angeschlossen :

Vom Tank $T_1$ zirkuliert über eine Pumpe $P_1$ und einen Kühler $K_1$ die zu entsalzende Lösung, die analog zur Literatur als Diluat D bezeichnet ist.

Vom Tank $T_2$ zirkuliert über eine Pumpe $P_2$ und einen Kühler $K_2$ die wässrige Lösung, welche die Salze aufnimmt und anreichert, die als Konzentrat K bezeichnet ist.

Vom Tank $T_3$ zirkuliert über eine Pumpe $P_3$ eine 2%-ige (Gewicht/Volumen) wässrige Lösung von Natriumsulfat, die als Elektrodenspüllösung EL bezeichnet ist.

Durch die Kühler $K_1$ und $K_2$ führt man die durch Reibung erzeugte Wärme ab und hält die Lösungen auf 25 °C.

Im Kreislauf des Diluats D ist nach dem Kühler $K_1$ eine Leitfähigkeitsmeßzelle LF eingebaut. Über einen Zweipunktregler R wird der Zulauf von aufkonzentriertem Ultrafiltrat UK reguliert.

Auf der Konzentratseite wird aus dem Tank $T_2$ kontinuierlich ein Teil der aufkonzentrierten Salzlösung KA abgeführt und über die Pumpe $P_5$ durch destilliertes Wasser W ersetzt. Außerdem findet im Zellpaket ein gewisser Wassertransport von der Diluatseite zur Konzentratseite statt.

Ein Teil des Diluats D wird nach der Leitfähigkeitsmessung in der Leitfähigkeitsmeßzelle LF laufend entnommen und über ein Sterilfilter SF als fertiges produkt PR im Tank $T_4$ bei 0 °C bis zur Weiterverarbeitung zur jeweiligen fertigen Arzneiform gelagert.

Zum Anfahren der Elektrodialyseanlage wird der 2 l fassende Tank $T_1$ mit 1,2 l eines in der oben beschriebenen Weise hergestellten konzentrierten Ultrafiltrats UK betchickt. In den gleichgroßen Tank $T_2$ legt man 1 l destilliertes Wasser und im 1 l fassenden Tank $T_3$ 750 ml einer 2%-igen $Na_2SO_4$-Lösung vor. Nachdem das Zellpaket und alle Leitungen gefüllt und entlüftet sind, setzt man die drei pumpen $P_1$ bis $P_3$ in Gang, die jeweils etwa 90 l/h fördern. An die beiden Elektrodenplatten (+ und -) wird ein Gleichstrom gelegt, wobei die Spannung so gewählt wird, daß auf den freien Membranquerschnitt ein Strom von etwa 20 mA/cm² fließt. Beim verwendeten Zellpaket mit einem freien Membranquerschnitt von 37 cm² fliessen somit insgesamt etwa 750 mA Strom. Die Anfangsspannung von etwa 16 V wird innerhalb 1 Stunde auf den dann stationären Wert von etwa 5,5 V für das aus sieben Membranpaaren bestehende Zellpaket zurückgeregelt.

Nach 10 Stunden ist die Leitfähigkeit auf einen Wert zwischen 45 und 50 mS/cm abgesunken. Der Regler R regelt nunmehr den Zulauf von weiterem konzentriertem Ultrafiltrat UK über eine Dosierpumpe $P_4$ derart, daß die Leitfähigkeit des Diluats zwischen 45 und 50 mS/cm bleibt, im Mittel auf etwa 100 ml/h.

Die Dosierpumpe $P_5$ wird auf 200 ml/h eingestellt, der Auslauf des Salzkonzentrats KA aus dem Tank $T_2$ beträgt etwa 220 ml/h.

Die unter mehrmaliger Wiederholung der obigen Dialyse unter Verwendung der beiden verschiedenen Ausgangskonzentrate Nr. 733 10 und Nr. 350 03 und Bildung der verschiedenen Elektrodialysate Nr. 12 und Nr. 13 sowie Nr. 14, Nr. 15, Nr. 16, Nr. 17, Nr. 18 und Nr. 19 erhaltenen Ergebnisse gehen zusammen mit den bei der Elektrodialyse angewandten Bedingungen von Temperatur, Stromdichte und Gleichspannung sowie den verschiedenen Analysedaten für die Ausgangtkonzentrate und die gewonnenen Elektrodialysate sowie den vergleichenden Daten über die jeweilige biologische Aktivität aus der folgenden Tabelle I hervor.

Die Versuchsergebnirse zeigen, daß sich unter Anwendung des erfindungsgemäßen Verfahrens in besonders eleganter und wirtschaftlicher Weise aus Kälberblut eine zellatmungsfördernde Wirkstofflösung herstellen läßt, die der nach den bekannten Verfahren erhältlichen entsprechenden Wirkstofflösung bezüglich ihrer biologischen Aktivität wenigstens gleichwertig und teilweise sogar überlegen ist.

Tabelle I

| | Dimension | Ausgangskonzentrat No. 733.10 | Elektrodialysat No. 12 | Elektrodialysat No. 13 |
|---|---|---|---|---|
| Trockengehalt | mg/ml | 212,1 | 103,0 | 108,7 |
| Dichte (20°C) | g/ml | 1,129 | 1,057 | 1,060 |
| Leitfähigkeit | mS/cm | 149 | 56 | 68 |
| Osmolarität | mOsm | 1053 ** | 404 *** | 497 *** |
| Aschegehalt | % Trockensubstanz | 66,25 | 33,90 | 41,80 |
| Gesamt-Stickstoff | % Trockensubstanz | 2,55 | 4,83 | 4,57 |
| Amino-Stickstoff | % Trockensubstanz | 0,65 | 1,23 | 1,25 |
| Chlorid | % Trockensubstanz | 35,88 | 15,74 | 19,31 |
| Natrium | % Trockensubstanz | 27,19 | 17,30 | 19,49 |
| Kalium | % Trockensubstanz | 4,34 | 1,59 | 2,14 |
| Glucose | % Trockensubstanz | 10,91 | 22,30 | 20,52 |
| Harnstoff | % Trockensubstanz | 2,15 | 3,59 | 3,43 |
| Gesamt-Phosphor | % Trockensubstanz | 0,78 | 1,52 | 1,39 |
| Lactat | % Trockensubstanz | 9,72 | 17,58 | 17,71 |
| Essigsäure | % Trockensubstanz | 1,83 | 3,45 | 3,18 |
| Fibroblasten-Aktivität | Wachstumsförderung gegenüber Standard-Kultur * | 1,6** | 1,5*** | 1,5*** |
| Warburg-Aktivität | % gegenüber Standard | -2 ** | +10 *** | +14 *** |
| Wundheilungsdauer gegen physiologische Kochsalzlösung | % Verkürzung | 9,9 ** | | 13,7/10,9 *** |
| Temperatur | °C | | 25 | 25 |
| Stromdichte | mA/cm² | | 50 | 50 |
| Gleichspannung pro Membran | V | | 0,7 | 1,0 |

* Alle Werte über 1,0 sind gut ; quantitative Abstufungen sind nicht mehr möglich.

** Diese Meßdaten beziehen sich auf ein Ultrafiltrat, das mit sterilem Wasser auf das Fünffache verdünnt worden ist.

*** Diese Meßdaten beziehen sich auf ein Elektrodialysat, das mit sterilem Wasser auf das Fünffache verdünnt worden ist.

Tabelle I (Fortsetzung)

| | Dimension | Ausgangskonzentrat Nr. 305.03 | Elektrodialysat Nr. 14 | Elektrodialysat Nr. 15 | Elektrodialysat Nr. 16 | Elektrodialysat Nr. 17 | Elektrodialysat Nr. 18 | Elektrodialysat Nr. 19 |
|---|---|---|---|---|---|---|---|---|
| Trockengehalt | mg/ml | 213,11 | 109,7 | 106,37 | 107,58 | 111,37 | 129,62 | 101,28 |
| Dichte (20°C) | g/ml | 1,129 | 1,060 | 1,057 | 1,058 | 1,060 | 1,073 | 1,052 |
| Leitfähigkeit | mS/cm | 145 | 56 | 50 | 55 | 56 | 40(6°C) | 46 |
| Osmolarität | mOsm | 1052** | 414*** | 378*** | 410*** | 428*** | 510*** | 355*** |
| Aschegehalt | % TS | 75,57 | 34,55 | 32,48 | 36,11 | 36,72 | 43,90 | 28,10 |
| Gesamt-Stickstoff | % TS | 2,73 | 5,59 | 6,26 | 5,61 | 5,87 | 5,13 | 6,16 |
| Amino-Stickstoff | % TS | 0,64 | 1,38 | 1,56 | 1,46 | 1,45 | 1,28 | 1,64 |
| Chlorid | % TS | 37,17 | 12,28 | 9,20 | 11,89 | 12,69 | 16,30 | 7,30 |
| Natrium | % TS | 24,79 | 18,03 | 16,99 | 18,23 | 18,36 | 20,40 | 17,30 |
| Kalium | % TS | 3,78 | 1,20 | 1,07 | 1,10 | 1,12 | 1,53 | 0,92 |
| Glucose | % TS | 3,87 | 8,65 | 9,81 | 9,06 | 9,09 | 7,95 | 10,28 |
| Harnstoff | % TS | 2,43 | 4,76 | 5,39 | 4,56 | 5,11 | 4,34 | 5,04 |
| Gesamt-Phosphor | % TS | 0,77 | 1,75 | 1,91 | 1,75 | 1,67 | 1,56 | 1,94 |
| Lactat | % TS | 9,78 | 20,62 | 22,72 | 21,29 | 21,63 | 18,32 | 22,60 |
| Essigsäure | % TS | 2,37 | 4,48 | 4,58 | 4,56 | 4,62 | 4,01 | 4,68 |
| Fibroblasten-Aktivität Wachstums-fördernd geg. Standard-Kultur* | % geg. Standard | 2,0** | 1,1*** | 1,2*** | 1,3*** | 0,7*** | 1,8*** | 2,0*** |
| Warburg-Aktivität | % geg. Standard | -6** | +5*** | +6*** | -7*** | +30*** | +9*** | +30*** |
| Wundheilungsdauer gegen physiologische Kochsalzlösung | % Verkürzung | 15,7** | | | | | | 9,0*** |
| Temperatur | °C | | 25 | 25 | 25 | 25 | 6 | 25 |
| Stromdichte | mA/cm² | | 50 | 50 | 22 | 50 | 50 | 20 |
| Gleichspannung pro Membran | V | | 1,0 | 1,2 | 0,8 | 1,2 | 1,3 | 0,4 |

TS = Trockensubstanz

* Alle Werte über 1,0 sind gut : quantitative Abstufungen sind nicht mehr möglich.

** Diese Meßdaten beziehen sich auf ein Ultrafiltrat, das mit sterilem Wasser auf das Fünffache verdünnt worden ist.

*** Diese Meßdaten beziehen sich auf ein Elektrodialysat, das mit sterilem Wasser auf das Fünffache verdünnt worden ist

EP 0 095 170 B2

**Patentansprüche**

1. Verfahren zur Gewinnung zellatmungsfördernder Wirkstoffe mit Molekulargewichten im Bereich von etwa 300 bis 8000 Dalton durch Defibrinieren von Kälberblut unmittelbar nach der Entnahme durch intensives Rühren und Abfiltreren des dabei gebildeten Fibrins, Hämolysieren der hierbei erhaltenen Lösung, Abtrennung der in der hämolysierten Lösung enthaltenen Proteine und Substanzen mit einem Molekulargewicht von über etwa 8000 Dalton mittels eines Membrantrennverfahrens, Einengung der hierdurch erhaltenen, von Proteinen und höhermolekularen Substanzen befreiten Lösung unter vermindertem druck bei einer 40 °C nicht übersteigenden Temperatur auf eine Dichte bei 20 °C im Bereich von 1,10 bis 1,15 g/ml und teilweise Abtrennung der im erhaltenen Konzentrat vorhandenen anorganischen Salze unter gegebenenfalls abschließender Verdünnung des erhaltenen Konzentrats zur Bildung einer isotonischer bis leicht hypertonischen Lösung mit einer Osmolarität im Bereich von 250 bis 550 mOsmol, dadurch gekennzeichnet, daß als Membrantrennverfahren eine kontinuierliche mehrstufige Ultrafiltration unter Verwendung von Membranen mit einer Molekulargewichtsausschlußgrenze von über etwa 8000 Dalton angewandt wird und die teilweise Abtrennung der anorganischen Salze durch Elektrodialyse mittel eines Zellpakets aus alternierend angeordneten Kationenund Anionenaustauschermembranen mit einer Membrandurchlässigkeit bis zu einem Molekulargewicht von etwa 300 Dalton und unter Anwendung eines Stromdichtebereichs von 5 bis 70 mA/cm$^2$ freiem Membranquerschnitt, vorzugsweise 20 bis 50 mA/cm$^2$ freiem Membranquerschnitt, einer Gleichspannung von 0,2 bis 2 V pro Membran, vorzugsweise 0,5 bis 1 V pro Membran, und eines Temperaturbereichs von 0 bis 30 °C durchgeführt wird.

2. Verfahren nach Anspruch 1 zur Gewinnung zellatmungsfördernder Wirkstoffe mit Molekulargewichten im Bereich: von etwa 400 bis 5000 Dalton, dadurch gekennzeichnet, daß bei der Ultrafiltration Membranen mit einer Molekulargewichtsausschlußgrenze von über etwa 5000 Dalton und bei der Elektrodialyse Membranen mit einer Durchlässigkeit bis zu einem Molekulargewicht von etwa 400 Dalton verwendet werden.

3. Verfahren nach einem der Ansprüche 1 oder 2, dadurch gekennzeichnet, daß man in die zwischen der ersten und der letzter Stufe der mehrstufigen Ultrafiltration liegenden Stufen jeweils eine solche Menge an Verdünnungsmittel einführt, daß hierdurch die aus dem jeweiligen Ultrafiltrationsmodul auslaufende Menge an Ultrafiltrat unter Beibehaltung eines etwa konstanten Niveaus an ultrazufiltrierender Blutlösung kompensiert wird.

4. Verfahren nach einem der Ansprüche 1 bis 3, dadurch gekennzeichnet, daß man in allen vor der letzten Stufe liegenden Stufen der mehrstufigen Ultrafiltration jeweils unter Beibehaltung eines etwa konstanten Niveaus an ultrazufiltrierender Blutlösung arbeitet.

5. Verfahren nach einem der Ansprüche 1 bis 4, dadurch gekennzeichnet, daß man die Elektrodialyse unter Bildung einer Lösung mit einer Leitfähigkeit im Bereich von 40 bis 75 mS/cm und mit einer Dichte bei 20 °C im Bereich von 1,05 bis 1,08 g/ml durchführt.

**Claims**

1. Process for obtaining cell respiratory stimulating active agents having a molecular weight in the range of about 300 to 8 000 Dalton by defibrination of calves blood immediately after taken by intensive stirring and filtering off of the fibrin thus formed, hemolysing the solution obtained thereby, separating the proteins and substances having a molecular weight of over about 8 000 Dalton comprised in the hemolysed solution by a membrane separation procedure, concentrating the resulting protein- and high molecular weight-freed solution under reduced pressure at a temperature not exceeding 40 °C to a concentration in the range of 1.10 to 1.15 g/ml at 20 °C and partially separating the inorganic salts from the resulting concentrated solution under optional final dilution of the obtained concentrate to form an isotonic to slightly hpertonic solution having an osmolarity in the range of 250 to 550 mOsmol, characterized in that the membrane separation procedure employed is a continuous multi-step ultrafiltration procedure employing membranes with a molecular weight exclusion limit of about over 8 000 Dalton and the partial separation of the inorganic salts is carried out by electrodialysis with the aid of a cell stack of alternately arranged cation- and anion-exchange membranes with a membrane permeability up to a molecular weight of about 300 Dalton, and under employment of a current density range of 5 to 70 mA/cm$^2$ free membrane cross-section, preferably 20 to 50 mA/cm$^2$ free membrane cross-section, a direct current of 0.2 to 2 V per membrane, preferably 0.5 to 1 V per membrane, and a temperature range of 0 to 30 °C.

2. Process according to claim 1, for obtaining cell respiratory stimulating active agents having a molecular weight in the range of about 400 to 5 000 Dalton, characterized in that in the ultrafiltration procedure membranes with a molecular weight exclusion limit of about over 5 000 Dalton, and in the electrodialysis membranes with

11

a permeability up to a molecular weight of about 400 Dalton are employed.

3. Process according to any of claims 1 or 2, characterized in that in the steps between the first and the last steps of the continuous multi-step ultrafiltration each such an amount of a dilution agent is added that the amount of ultrafiltrate flowing from the individual ultrafiltration module is compensated thereby under maintaining an about constant level of blood solution to be ultrafiltrated again in the next step.

4. Process according to any of claims 1 to 3, characterized in that in all but the last step of the continuous multi-step ultrafiltration an about constant level of the blood solution to be ultrafiltrated is maintained.

5. Process according to any of claims 1 to 4, characterized in that the exlectrodialysis is carried out to form a solution having a conductivity in the range of 40 to 75 mS/cm and a density at 20 °C in the range of 1.05 to 1.08 g/ml.

**Revendications**

1. Procédé pour l'obtention de substances actives accélérant la respiration cellulaire ayant des poids moléculaires dans un intervalle d'environ 300 à 8 000 dalton par défibrination du sang de veau directement après prélèvement, par agitation intense et élimination par filtration de la fibrine formée, hémolyse de la solution ainsi obtenue, séparation des protéines et substances de poids moléculaires de plus de 8 000 dalton contenues dans la solution hémolysée par un procédé de séparation par membrane, concentration de la solution ainsi obtenue, débarrassée de protéines et de substances de haut poids moléculaire, sous pression réduite à une témpérature ne dépassant pas 40 °C, à une densité à 20 °C comprise entre 1,10 et 1,15 g/ml et séparation partielle des sels inorganiques présents dans le concentré obtenu, suivie éventuellement de dilution du concentré obtenu pour la formation d'une solution isotonique à faiblement hypertonique ayant une osmolarité comprise entre 250 et 550 mOsmol, caractérisé en ce que l'on utilise comme procédé de séparation par membrane une ultrafiltration multiétage continue avec utilisation de membranes ayant une limite d'exclusion de poids moléculaire de plus d'environ 8 000 dalton et on effectue la séparation partielle des sels inorganiques par électrodialyse au moyen d'une batterie de cellules à membranes échangeuses de cations et échangeuses d'anions disposées alternativement, ayant une perméabilité de membrane jusqu'à un poids moléculaire d'environ 300 dalton, et avec utilisation d'une tensité de courant dans l'intervalle de 5 à 70 mA/cm² de section droite libre de membrane, de préférence de 20 à 50 mA/cm² de section droite libre de membrane, d'une tension continue de 0,2 à 2 V par membrane, de préférence de 0,5 à 1 V par membrane et d'une température dans l'intervalle de 0 à 30 °C.

2. Procédé selon la revendication 1 pour l'obtention de substances actives accélérant la respiration cellulaire ayant des poids moléculaires compris entre 400 et 5 000 dalton, caractérisé en ce que l'on utilise dans l'ultrafiltration des membranes ayant une limite d'exclusion de poids moléculaire de plus d'environ 5 000 dalton et dans l'électrodialyse des membranes ayant une perméabilité jusqu'à un poids moléculaire d'environ 400 dalton.

3. Procédé selon l'une quelconque des revendications 1 ou 2, caractérisé en ce que dans les étapes comprises entre la première et la dernière étape de l'ultrafiltration multi-étage, on introduit une quantité de diluant telle que la quantité d'ultrafiltrat s'écoulant de chaque module d'ultrafiltration soit ansi compensée avec maintien d'un niveau à peu près constant de la solution de sang à ultrafiltrer.

4. Procédé selon l'une quelconque des revendications 1 à 3, caractérisé en ce que dans toutes les étapes avant la dernière étape de l'ultrafiltration multi-étage, on opère chaque fois avec maintien d'un niveau à peu près constant de la solution de sang à ultrafiltrer.

5. Procédé selon l'une quelconque des revendications 1 à 4, caractérisé en ce qu'on effectue l'électrodialyse avec formation d'une solution ayant une conductivité dans l'intervalle de 40 à 75 mS/cm et une densité à 20 °C dans l'intervalle de 1,05 à 1,08 g/ml.

Fig. 1

Stufe 1   Stufe 2   Stufe 3   Stufe 4

AB

UF

KL

B

$K_1$  $K_2$  $K_3$  $K_4$

$U_1$  $U_2$  $U_3$  $U_4$

$T_1$  $T_2$  $T_3$  $T_4$

$P_5$  $P_6$  $P_7$

$P_1$  $P_2$  $P_3$  $P_4$

$P_8$  $P_9$  $P_{10}$

13

Fig.2

EP 0 095 170 B2